# EUROPÄISCHE PATENTANMELDUNG

(11) **EP 1 842 571 A2**
(43) Veröffentlichungstag der Anmeldung: **10.10.2007**
(21) Anmeldenummer: 07105579.2
(22) Anmeldetag: 03.04.2007
(51) Int. Cl.: A61N 5/06

(54) **Infrarotleuchte**

(30) Priorität: 03.04.2006 DE 202006005347 U
(71) Anmelder: Efbe Elektrogeräte GmbH, 07422 Bad Blankenburg (DE)
(72) Erfinder: HEINZE, Bernd, 07426, Königsee (DE)
(74) Vertreter: Reitstötter - Kinzebach

(57) **Zusammenfassung**

Die vorliegende Erfindung betrifft eine Infrarotleuchte (10) mit einem im Wesentlichen trogförmigen Leuchtengehäuse (11) zur Aufnahme einer Infrarotlichtquelle, in welchem eine stirnseitige Strahlungsaustrittsöffnung (14) ausgespart ist, und einem mit dem Leuchtengehäuse (11) verbundenen Standfuß (15, 15'), die dadurch gekennzeichnet ist, dass das Leuchtengehäuse (11) zumindest teilweise von einem Schutzmantel (20) umgeben ist, wobei zwischen einer Außenfläche (21) des Leuchtengehäuses (11) und einer Innenfläche (22) des Schutzmantels (20) ein Luftspalt (23) definiert ist. Damit wird erreicht, dass der äußere Schutzmantel auch nach längerem Betrieb deutlich kühler als das innere Leuchtengehäuse selbst bleibt.

## Beschreibung

Die vorliegende Erfindung betrifft eine Infrarotleuchte mit einem im Wesentlichen trogförmigen Leuchtengehäuse zur Aufnahme einer Infrarotlichtquelle, in welchem eine stirnseitige Strahlenaustrittsöffnung ausgespart ist, und einem mit dem Leuchtengehäuse verbundenen Standfuß.

Derartige Infrarotleuchten, die auf Grund der typischerweise mit einem sichtbares Licht weitgehend blockierende, roten Filter versehenen Infrarotlichtquellen auch als Rotlichtlampen bezeichnet werden, sind im Stand der Technik bekannt, und werden vorzugsweise zur medizinischen Wärme- beziehungsweise Infrarottherapie eingesetzt. Die von den Infrarotleuchten emittierte Wärmestrahlung kann tief in Gewebe und Muskeln eindringen und fördert auf Grund der damit verbundenen gefäßerweiternden Wirkung die Durchblutung des bestrahlten Areals. Infrarottherapie wirkt besonders vorteilhaft bei der Schmerzbekämpfung, insbesondere bei der Linderung von Muskelverspannungen und Muskelkrämpfen und bei der Beschleunigung der Heilung von entzündlichen Prozessen, wie beispielsweise von Nebenhöhlenentzündungen. Auch bei der therapeutischen und kosmetischen Behandlung der Haut wird die Bestrahlung mit Infrarotlicht erfolgreich eingesetzt.

Als Infrarotlichtquellen werden elektrische Lichtquellen verwendet, die einen möglichst hohen Infrarotanteil besitzen. Zusätzlich wird häufig das im sichtbaren Spektralbereich emittierte Licht durch geeignete Filter absorbiert und somit ebenfalls in Wärme umgewandelt. Obwohl die typischerweise eingesetzten Infrarotlichtquellen meist verspiegelt und so ausgelegt sind, dass ein Großteil der erzeugten Wärmestrahlung gerichtet über die stirnseitige Strahlenaustrittsöffnung des Leuchtengehäuses emittiert werden kann, ist eine Erwärmung des Leuchtengehäuses selbst im Betrieb praktisch unvermeidlich. Dies erschwert die Einstellung und Handhabung der bereits im Betrieb befindlichen Infrarotleuchte durch den Benutzer erheblich.

Der vorliegenden Erfindung liegt daher das technische Problem zu Grunde, eine Infrarotleuchte der eingangs beschriebenen Art anzugeben, welche auch im Betrieb einfach und bequem handhabbar ist und insbesondere auch bei längerer Betriebsdauer vom Benutzer noch problemlos neu orientiert werden kann, ohne dass der Benutzer dabei mit heißen Flächen des Leuchtengehäuses in Kontakt kommt.

Zur Lösung dieses technischen Problems schlägt die Erfindung vor, das Leuchtengehäuse zumindest in einem Teilbereich doppelwandig auszubilden, wobei zwischen den beiden Wänden des Gehäuses eine wärmeisolierende Luftschicht vorhanden ist.

Gegenstand der Erfindung ist demnach eine Infrarotleuchte mit einem im Wesentlichen trogförmigen Leuchtengehäuse zur Aufnahme einer Infrarotlichtquelle, in welchem eine stirnseitige Strahlenaustrittsöffnung ausgespart ist, und einem mit dem Leuchtengehäuse verbundenen Standfuß, die dadurch gekennzeichnet ist, dass das Leuchtengehäuse zumindest teilweise von einem Schutzmantel umgeben ist, wobei zwischen einer Außenfläche des Leuchtengehäuses und einer Innenfläche des Schutzmantels ein als Wärmeisolator dienender Luftspalt definiert ist. Bei der erfindungsgemäßen Infrarotleuchte ist demnach der äußere Schutzmantel auch nach längerem Betrieb deutlich kühler als das innere Leuchtengehäuse selbst. Der Benutzer kann daher die Infrarotleuchte problemlos am Schutzmantel greifen und nach seinen Wünschen einstellen und ausrichten.

Der Kühleffekt wird begünstigt, wenn, wie bei einer bevorzugten Ausführungsform der erfindungsgemäßen Infrarotleuchte vorgesehen, in dem Schutzmantel mehrere Öffnungen ausgespart sind, durch die erwärmte Luft aus dem Luftspalt austreten und kühle Luft aus der Umgebung in den Luftspalt eindringen kann. Selbstverständlich kann zu diesem Zweck in dem durch das Leuchtengehäuse und den Schutzmantel definierten Luftspalt ein Fördermittel, beispielsweise ein elektrisch betriebener Ventilator angeordnet sein. Es erweist sich jedoch, dass für herkömmliche, insbesondere im Haushalt vom Privatanwender eingesetzte Infrarotleuchten mit einer typischen Leistung zwischen 50 und 200 W, die durch die Erwärmung der Luft im Luftspalt initiierte natürliche Konvektion für eine effektive Kühlung ausreicht, so dass kein zusätzlicher Ventilator zur Umwälzung der Luft erforderlich ist.

Derartige Infrarotleuchten für den Haushaltsgebrauch weisen üblicherweise einen kleinen Standfuß auf, der eine Höhe besitzt, welche es erlaubt, die Leuchte bequem auf einem Tisch zu platzieren. Die Leuchte ist dann üblicherweise leicht nach oben gerichtet orientiert, um beispielsweise den Gesichtsbereich des Benutzers zu bestrahlen. Daher ist es vorteilhaft, einerseits Öffnungen im stirnseitigen Endbereich des Schutzmantels anzuordnen, durch welche die aufsteigende erwärmte Luft aus dem Luftspalt in die Umgebung entweichen kann. Diese ersten Austrittsöffnungen können beispielsweise als im Wesentlichen axial orientierte, d.h. im wesentlichen parallel zur Längsachse der Leuchte verlaufende Schlitze ausgebildet sein, die am Umfang des stirnseitigen Endbereichs des Schutzmantels ausgespart sind. Ebenso erweist es sich als vorteilhaft, in einem, von dem stirnseitigen Endbereich abgewandten Abschnitt des Schutzmantels eine zweite Gruppe von Öffnungen anzuordnen, durch welche kühlere Luft aus der Umgebung in den Luftspalt eindringen kann. Die zweiten Öffnungen können beispielsweise radial zur Längsachse der Infrarotleuchte orientiert und in einem rückseitigen Endbereich eines ebenfalls trogförmig ausgebildeten Schutzmantels ausgespart sein. Durch das Zusammenspiel der ersten und zweiten Öffnungen wird im Betrieb der Infrarotleuchte auf Grund des durch die Erwärmung der Luft im Luftspalt resultierenden Kamineffekts eine effektive und dauerhafte Kühlung bewirkt, so dass sich der Schutzmantel auch bei längerem Betrieb nicht oder nur unwesentlich erwärmt. Die ersten und zweiten Öffnungen sind dabei vorzugsweise im Wesentlichen gleichmäßig um die Längsachse der Leuchte herum verteilt.

Der Schutzmantel besteht vorzugsweise aus einem Material mit geringer Wärmeleitfähigkeit, beispielsweise einem Kunststoffmaterial. Besonders vorteilhaft besteht der Schutzmantel aus einem transparenten Material, wie beispielsweise Polycarbonat.

Gemäß einer besonders bevorzugten Ausführungsform ist das Leuchtengehäuse schwenkbar an dem Standfuß angelenkt. Dazu kann der Standfuß beispielsweise einen U-förmigen Träger aufweisen, dessen beiden Arme durch zwei der ersten axial orientierten, schlitzförmigen Öffnungen des Schutzmantels greifen und an einem Drehgelenk des Leuchtengehäuses schwenkbar montiert sind.

Besonders vorteilhaft weist die erfindungsgemäße Infrarotleuchte eine mechanische oder elektrische Zeitschaltuhr auf, welche es ermöglicht, die Behandlungsdauer beispielsweise gemäß einer von einem Arzt verordneten Behandlungsdauer einzustellen.

Gemäß einer einfachen Variante ist die Zeitschaltuhr als Drehschalter ausgebildet. Die Zeitschaltuhr kann außerdem ein numerisches Display umfassen, welches die eingestellte Gesamtbehandlungsdauer in Leuchtziffern, beispielsweise in Form einer 7-Segmentanzeige, darstellt. Die Einstellung der Gesamtbehandlungsdauer kann dabei wiederum über einen Drehknopf oder über Druckknöpfe erfolgen, mit denen die Behandlungsdauer beispielsweise bei jeder Betätigung des Druckknopfes um einen festen Wert verlängert oder verkürzt wird. Die Anzeige kann die Gesamtbehandlungsdauer und/oder, im laufenden Betrieb, die Restbehandlungsdauer anzeigen. Es kann beispielsweise auch ein Druckknopf vorgesehen sein, mit dem zwischen der Gesamtbehandlungsdauer und der Restbehandlungsdauer hin- und hergeschaltet werden kann. Insbesondere wenn in der numerischen Anzeige nur die Gesamtbehandlungsdauer angezeigt wird, kann man eine aus mehreren lichtemittierenden Dioden (LEDs) bestehende Diodenleiste vorsehen, wobei zu Beginn der Behandlung die gesamte Leiste leuchtet und mit fortschreitender Behandlungsdauer eine LED nach der anderen ausgeschaltet wird, so dass der Benutzer einen leicht ablesbaren optischen Hinweis auf die restliche Behandlungsdauer erhält.

Die Erfindung wird im Folgenden an Hand eines bevorzugten Ausführungsbeispiels näher erläutert.

In der Zeichnung zeigt:
- Figur 1: eine perspektivische Darstellung der erfindungsgemäßen Infrarotleuchte, wobei zur besseren Übersichtlichkeit die Infrarotlichtquelle selbst weggelassen wurde;
- Figur 2: eine Seitenansicht der Infrarotleuchte der Figur 1;
- Figur 3: eine Rückansicht der Infrarotleuchte der Figur 1; und
- Figur 4: eine Variante des Fußes der Infrarotleuchte der Figur 1 in vergrößerter Darstellung.

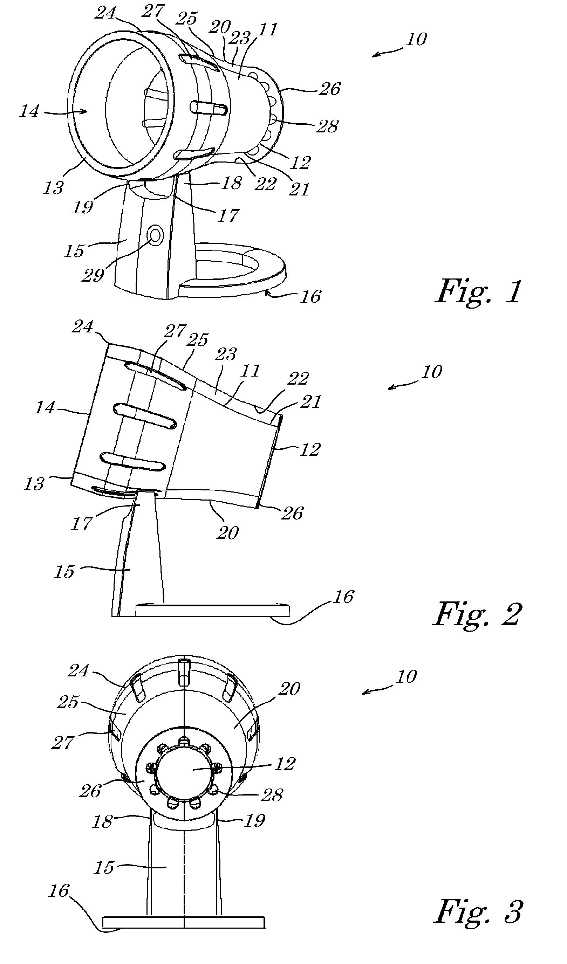

Figur 1 zeigt eine insgesamt mit der Bezugsziffer 10 bezeichnete Infrarotleuchte, die ein im Wesentlichen trogförmiges Leuchtengehäuse 11 aufweist, in welchem eine (in den Figuren der besseren Übersichtlichkeit halber nicht dargestellte, an sich bekannte) Infrarotlichtquelle montiert werden kann. Dazu weist die Infrarotleuchte üblicherweise an sich bekannte elektrische Anschlussmittel zur Versorgung der Infrarotlichtquelle mit elektrischer Energie auf. Beispielsweise kann im Bereich des Bodens 12 des Gehäuses 11 eine (nicht dargestellte) Schraubfassung angeordnet, in welche die Infrarotlichtquelle mit ihrem Lampensockel eingeschraubt werden kann, was ein bequemes Auswechseln der Infrarotlichtquelle im Fall eines Defekts erlaubt. Im stirnseitigen Bereich 13 des trogförmigen Leuchtengehäuses 11 ist eine Öffnung 14 ausgespart, durch welche die Infrarotlichtquelle in das Gehäuse eingebaut werden kann. Die Öffnung 14 dient gleichzeitig als Austrittsöffnung für die Infrarotstrahlung der Infrarotlichtquelle. Das Leuchtengehäuse 11 ist auf einem Standfuß 15 montiert, dessen Unterseite 16 mit einer rutschfesten Auflage, beispielsweise einer Gummifläche, beschichtet ist. Im dargestellten Beispiel weist der Standfuß 15 einen U-förmigen Träger 17 auf, dessen beiden Arme 18, 19 an (hier nicht detaillierter dargestellten) Drehgelenken des Leuchtengehäuses 11 schwenkbar montiert sind (vergleiche insbesondere auch die Rückansicht der Figur 3).

Wie man insbesondere der Seitenansicht der Figur 2 entnehmen kann, ist das Leuchtengehäuse 11 von einem ebenfalls im Wesentlichen trogförmigen Schutzmantel 20 umgeben. Zwischen der Außenfläche 21 des Leuchtengehäuses 11 und der Innenfläche 22 des Schutzmantels 20 ist ein als Wärmeisolator dienender Luftspalt 23 definiert.

Im dargestellten Beispiel besteht der Schutzmantel 20 aus zwei verschiedenen Materialien. Im Stirnbereich 13 des Leuchtengehäuses 11 setzt sich der Schutzmantel 20 des Leuchtengehäuses 11 als L-förmig zurückgeschlagener Abschnitt 24 fort. Der L-förmig zurückgeschlagene Abschnitt 24 bildet daher einen die Austrittsöffnung 14 umgebenden Kranz. Zur Rückseite der Leuchte 10 hin schließt sich an den L-förmig zurückgeschlagenen Abschnitt 24 ein Abschnitt 25 des Schutzmantels 20 aus transparentem Material an. Der Abschnitt 25 weist einen senkrecht zur Längsachse der Infrarotleuchte 10 orientierten Endbereich 26 auf, welcher die Verbindung mit dem Leuchtengehäuse 11 im hinteren Abschnitt der Leuchte wiederherstellt.

Im Stirnbereich des Schutzmantels 20 sind im Wesentlichen parallel zur Längsachse der Infrarotleuchte orientierte, schlitzartige Öffnungen 27 ausgespart, durch welche heiße Luft aus dem Luftspalt 23 in die Umgebung entweichen kann. Im Endbereich 26 des Schutzmantels 20 sind dem gegenüber radial zur Längsachse orientierte Öffnungen 28 ausgespart, durch die kühle Luft von außen in den Luftspalt 23 nachströmen kann. Durch den daraus resultierenden Kamineffekt wird eine effektive Kühlung der erfindungsgemäßen Infrarotleuchte bewirkt.

Schließlich ist mit der Bezugsziffer 29 eine am Fuß 15 der Infrarotleuchte 10 angeordnete Zeitschaltuhr angedeutet. In der in Figur 1 dargestellten Variante ist die Zeitschaltuhr schematisch als Drehschalter 29 dargestellt, welcher die Einstellung der Gesamtbehandlungsdauer ermöglicht. Der Drehschalter kann beispielsweise eine einrastende Nullstellung aufweisen, so dass mit der Einstellung der Behandlungsdauer auch das Ein- und Ausschalten der Infrarotleuchte bewirkt werden kann.

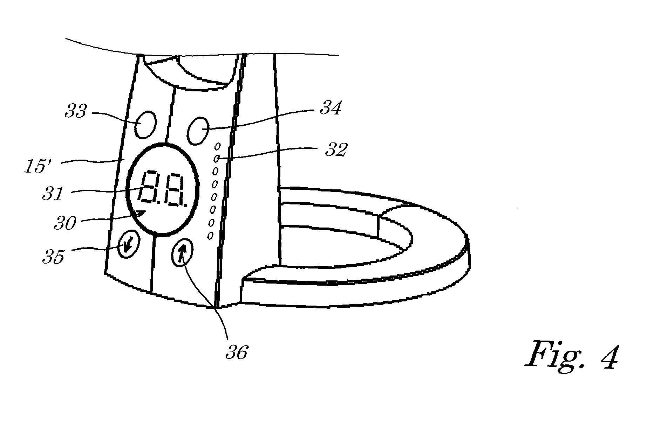

In Figur 4 zeigt eine Variante des Fußes 15' der Infrarotleichte der Figur 1 in einer vergrößerten Ausschnittsdarstellung, wobei die Leuchte selbst nicht dargestellt ist. Bei dieser Variante weist die Zeitschaltuhr 30 ein numerisches Display 31 mit 7-Segment-Anzeige zur Anzeige der eingestellten Gesamtbehandlungsdauer und eine aus mehreren lichtemittierenden Dioden bestehende LED-Leiste 32 zur Anzeige der Restbehandlungsdauer auf. Im dargestellten Beispiel sind über dem Display 31 zwei Druckschalter 33 und 34 zum Ein- bzw. Ausschalten der Infrarotleuchte vorgesehen, während Druckschalter 35 bzw. 36 dazu dienen, durch mehrfache Betätigung die in der numerischen Display 31 dargestellte Gesamtbehandlungsdauer zu verringern (Druckschalter 35) oder zu vergrößern (Druckschalter 36). Bei der dargestellten Variante wird im numerischen Display 31 also stets die Gesamtbehandlungsdauer angezeigt, während im laufenden Betrieb die Anzahl der in der LED-Leiste 32 jeweils leuchtenden LEDs proportional zur momentanen Restbehandlungsdauer ist. Somit erhält der Benutzer stets einen Hinweis auf die verbleibende Restbehandlungsdauer. Selbstverständlich kann man auch vorsehen, dass das numerische Display 31 zwischen der Gesamtbehandlungsdauer und der momentanen Restbehandlungsdauer umschaltbar ist oder sogar in vorgegebenen Intervallen zwischen den beiden Zeitanzeigen hin- und herwechselt.

## Patentansprüche

1. Infrarotleuchte mit einem im Wesentlichen trogförmigen Leuchtengehäuse (11) zur Aufnahme einer Infrarotlichtquelle, in welchem eine stirnseitige Strahlungsaustrittsöffnung (14) ausgespart ist, und einem mit dem Leuchtengehäuse (11) verbundenen Standfuß (15, 15'),
**dadurch gekennzeichnet,**
**dass** das Leuchtengehäuse (11) zumindest teilweise von einem Schutzmantel (20) umgeben ist, wobei zwischen einer Außenfläche (21) des Leuchtengehäuses (11) und einer Innenfläche (22) des Schutzmantels (20) ein Luftspalt (23) definiert ist.

2. Infrarotleuchte gemäß Anspruch 1, **dadurch gekennzeichnet, dass** in dem Schutzmantel (20) mehrere Öffnungen (27,28) ausgespart sind.

3. Infrarotleuchte gemäß Anspruch 2, **dadurch gekennzeichnet, dass** erste Öffnungen (27) im stirnseitigen Endbereich (13) des Schutzmantel (20) angeordnet sind.

4. Infrarotleuchte gemäß Anspruch 3, **dadurch gekennzeichnet, dass** zweite Öffnungen (28) in einem, von dem stirnseitigen Endbereich (13) abgewandten Abschnitt (26) des Schutzmantel (20) angeordnet sind.

5. Infrarotleuchte gemäß Anspruch 4, **dadurch gekennzeichnet, dass** die ersten und zweiten Öffnungen (27,28) im wesentlichen gleichmäßig um die Längsachse der Leuchte (10) verteilt sind.

6. Infrarotleuchte gemäß einem der Ansprüche 1 bis 5, **dadurch gekennzeichnet, dass** der Schutzmantel (20) zumindest teilweise aus einem transparenten Material besteht.

7. Infrarotleuchte gemäß einem der Ansprüche 1 bis 5, **dadurch gekennzeichnet, dass** das Leuchtengehäuse (11) schwenkbar an dem Standfuß (15) angelenkt ist.

8. Infrarotleuchte gemäß Anspruch 7 in Verbindung mit einem der Ansprüche 3 bis 6, **dadurch gekennzeichnet, dass** der Standfuß (15) einen U-förmigen Träger (17) umfasst, dessen beiden Arme (18,19) durch zwei der ersten Öffnungen (27) des Schutzmantels (20) greifen und an einem Drehgelenk des Leuchtengehäuses (11) schwenkbar montiert sind.

9. Infrarotleuchte gemäß einem der Ansprüche 1 bis 8, **dadurch gekennzeichnet, dass** eine Zeitschaltuhr (29;30) zur Einstellung der Betriebsdauer der Leuchte vorgesehen ist.

10. Infrarotleuchte gemäß Anspruch 9, **dadurch gekennzeichnet, dass** die Zeitschaltuhr (30) eine numerisches Display (31) umfasst.

11. Infrarotleuchte gemäß einem der Ansprüche 9 oder 10, **dadurch gekennzeichnet, dass** die Zeitschaltuhr (30) eine LED-Leiste (32) umfasst.
